# FASCICULE DE BREVET EUROPEEN

(11) **EP 0 353 150 B1**
(45) Date de publication et mention de la délivrance du brevet: **31.08.1994**
(21) Numéro de dépôt: 89402124.5
(22) Date de dépôt: 26.07.1989
(51) Int. Cl.: C12P 21/02, C07K 13/00, A61K 37/02, C07K 3/28

(54) **IL2 humaine recombinante non glycosylée sous forme réduite, son procédé d'obtention et son application comme médicament**
Rekombinantes, unglycosiliertes Human-IL2 in reduzierter Form, Herstellungsprozess und medizinische Anwendung
Recombinant non-glycosylated human IL2 in reduced form, process for its production and its medical use

(30) Priorité: 28.07.1988 FR 8810184
(43) Date de publication de la demande: 31.01.1990
(73) Titulaire: ROUSSEL-UCLAF, 75007 Paris (FR)
(72) Inventeur: Lando, Danielle, F-75020 Paris (FR); Riberon, Philippe, F-94130 Nogent sur Marne (FR); Abecassis, Pierre Yves, F-75020 Paris (FR)
(74) Mandataire: Bourgouin, André

(56) Documents cités:
- EP-A- 0 118 617
- EP-A- 0 145 390
- THE JOURNAL OF BIOLOGICAL CHEMISTRY, vol. 262, no. 12, 25 avril 1987, pages 5723-5731, The American Society of Biological Chemists, Inc., Baltimore, US; G. JU et al.: "Structure-function analysis of human interleukin-2"
- BIOCHEMISTRY, vol. 26, no. 11, 2 juin 1987, pages 3129-3134, American Chemical Society, Washington, D.C., US; T. TSUJI et al.: "Characterization of disulfidebonds in recombinant proteins: Reduced human interleukin 2 in inclusion bodies and its oxidative refolding"

## Description

La présente invention concerne une Interleukine 2 humaine recombinante, non glycosylée (notée r-hIL₂), sous forme réduite, biologiquement active, son procédé d'obtention et son application comme médicament.

L'IL₂ naturelle humaine qui est une lymphokine stimulant la prolifération de cellules T activées, possède 3 Cystèines localisées en position 58, 105 et 125 de la séquence en acides aminés de la protéine. Les cystéines en 58 et 105 sont réunies par un pont disulfure, tandis que la cystéine en 125 a un groupement sulfhydryle libre (Robb,RJ et al. Proc.Natl. Acad.Sci.USA (1984) 81 6486-6490).

Des procédés de préparation de l'IL₂ humaine, d'alléles ou de dérivés, par la technologie de l'ADN recombinant, sont décrits. Par exemple, Taniguchi,T et al. Nature (1983) 302 305-310 et Devos,R et al. Nucleic Acids Research (1983) 11 4307-4323, ont décrit le clonage du gêne de l'IL₂ humaine et son expression dans des microorganismes, et Ju,C et al. J.Biol.Chem (1987) 262 5723-5731 ont obtenu l'expression de dérivés recombinants de l'IL₂. Il est d'autre part connu que, lorsque l'IL₂ est accumulée dans le microorganisme à l'état de granules insolubles, elle se trouve sous forme réduite contenant 3 groupes thiols et est dépourvue d'activité (demande de brevet japonais J6 1257931). Il était donc admis que pour disposer d'une IL₂ active il fallait procéder à l'oxydation de la protéine réduite accumulée dans les granules. Pour ce faire, après dissolution de la protéine en milieu dénaturant, la formation du pont disulfure convenable 58-105 qui nécessite une renaturation, était effectuée en milieu oxydant contrôlé. Différentes méthodes sont décrites telles que l'oxydation par l'oxygène seul (autooxydation à l'air) ou et en présence d'ions cuivriques, ou d'un oxydant faible tel qu'un thiol ou encore par un mélange thiol-disulfure (Tsuji,T et al. Biochemistry (1987) 26 3129-3134).

Après renaturation oxydative, une purification par chromatographie est nécessaire pour éliminer les produits d'oxydation correspondant à la formation de ponts intramoléculaires isomères 58-125 et 105-125 ainsi que de ponts intermoléculaires dont on a montré qu'ils sont inactifs et peuvent être séparés par chromatographie en phase inversée selon Wang,A et al. Science (1984) 224 1431-1433 ou Browing,JL et al. Anal Biochem (1986) 155 123-128).

L'obtention de l'IL₂ recombinante oxydée homogène ayant l'activité biologique convenable en partant de la protéine accumulée sous forme de granules, pose donc des problèmes techniques, quelque soit le procédé suivi, car elle nécessite plusieurs étapes de purification ce qui conduit à un abaissement du rendement en produit cherché.

La présente demande concerne une nouvelle IL₂ humaine recombinante non glycosylée active, sous forme réduite et un procédé de préparation de ce produit qui ne nécessite pas de stade de réoxydation pour obtenir un produit manifestant l'activité biologique de l'IL₂.

Un aspect de l'invention concerne l'Interleukine 2 humaine recombinante non glycosylée ayant d'une part la séquence en acides aminés suivante : 
X-







ainsi que les allèles ou dèrivés de cette séquence dans laquelle X représente une méthionine ou un atome d'hydrogène et dont les trois cystèines en position 58, 105 et 125 sont sous la forme réduite, d'autre part manifestant une activité biologique comparable à celle de l'IL₂ oxydée ayant la même séquence et comportant un pont disulfure en position 58-105.

Par allèles et dérivés, on inclut les séquences modifiées par substitution, délétion ou addition de un ou plusieurs acides aminés autres que les cystéines 58, 105, 125 pour autant que ces produits conservent l'activité biologique caractéristique de l'IL₂ réduite. L'obtention de telles modifications est bien connue dans la méthode de l'ADN recombinant, par exemple par les techniques de mutagénèse dirigée qui ont été passées en revue par Lather, RF et Lecoq, JP dans Genetic Engineering Academic Press (1983) 31-50 ou Smith, M et Gillam, S Genetic Engineering Principles and methods Plenum Press (1981) 3 1-32. Par forme réduite, on entend que les restes cystéines que contient l'IL₂ renferment un groupement sulfhydryle libre dont la détermination est faite, par exemple, par spectrophotométrie avec la dithiodipyridine comme réactif des thiols. L'activité biologique de la forme réduite concernée par l'invention est déterminée, comparativement à celle de la forme oxydée correspondante contenant le pont disulfure 58-105, par mesure de la prolifération de lignées cellulaires leucémiques de souris dépendantes de l'IL₂ CTLL-2, avec un test colorimétrique au sel de tétrazolium (Mossmann,T J. Immunol Meth.(1983) 65 55-63).

L'invention concerne aussi une IL₂ humaine recombinante non glycosylée ayant d'une part la séquence en acides aminés suivante : 
X-







ainsi que les allèles ou dérivés de cette séquence dans laquelle X représente une méthionine ou un atome d'hydrogène et dont les trois cystéines en position 58, 105 et 125 sont sous la forme réduite, d'autre part manifestant une activité biologique d'au moins 0.5 x 10⁷ U/mg. L'unité d'activité IL₂ est définie comme la quantité qui produit 50 % de la réponse maximum dans le test. On utilise comme standard un échantillon "Biological Response Modifier Program (BRMP) reference reagent human IL₂(Jurkat)" fourni par National Cancer Institute (NCI).

L'invention concerne plus précisément l'interleukine 2 humaine recombinante non glycosylée ayant la séquence en acides aminés suivante : 
X-







dans laquelle X représente une méthionine ou un atome d'hydrogène et dont les trois cystéines en position 58, 105 et 125 sont sous la forme réduite et manifestant une activité biologique comparable à celle de l'IL₂ humaine native. Par activité comparable, on entend la même activité spécifique que celle de l'IL₂ naturelle isolée de cellules leucémiques Jurkat c'est à dire de 1.3 x 10⁷ U/mg (référence BRMP), ou une activité différente au maximum de 25 % de cette activité spécifique. La séquence de l'IL₂ réduite concernée par l'invention a éventuellement une méthionine N-terminale supplémentaire selon le microorganisme transformé, tel que E. coli, dans lequel elle est exprimée. Dans un mode préféré d'exécution de l'invention, on préfère la séquence comportant une méthionine, mais on peut également utiliser un mélange de produit comportant une méthionine avec du produit ne comportant pas de méthionine ou le produit dépourvu de méthionine.

Un autre aspect de l'invention concerne un procédé de préparation de l'IL₂ humaine recombinante non glycosylée réduite comprenant d'abord l'extraction de l'IL₂ accumulée sous forme de granules dans un microorganisme transformé par solubilisation en milieu réducteur à l'aide d'un agent chaotrope, puis la purification par précipitation suivie d'une chromatographie liquide à haute performance en phase inversée avec un éluant acide, et caractérisé en ce que :
a) le cas échéant l'on soumet la fraction principale éluée de la dite chromatographie à un stade de refroidissement à une température de l'ordre de -20¤ C, puis à une séparation de la phase aqueuse que
b) l'on dilue en milieu acide, puis chromatographie sur une autre colonne de chromatographie liquide à haute performance en phase inversée en milieu acide et isole la dite IL₂.

L'IL₂ recombinante produite sous forme de granules, grâce à un taux d'expression élevé, par un microorganisme transformé tel que E.coli, peut être, par des méthodes bien connues, solubilisée à l'aide d'une solution concentrée d'un agent chaotrope telle qu'une solution de sel de guanidine 6 à 8 M, puis purifiée par chromatographie liquide à haute performance en phase inversée (noté dans ce qui suit RP-HPLC) avec des supports disponibles dans le commerce, de préférence des silices greffées telles que C3, C4, C8 ou C18, avec un éluant acide ayant un pH compris de 1 à 4. L'IL₂ peut être éluée de la colonne à l'aide d'un système de gradient comprenant un acide organique tel que l'acide acétique ou l'acide trifluoroacétique (noté dans ce qui suit TFA) et un solvant organique tel que l'acétonitrile. La fraction principale dont l'élution est détectée par spectrophotométrie à 280 nm est la matière première pour le stade de refroidissement éventuel qui fait partie du procédé de la présente invention. Par refroidissement on entend que la dite fraction principale recueillie à température ambiante est placée dans un environnement de température de l'ordre de -20¤ C qui permet la formation progressive d'une phase aqueuse solide dont on peut éliminer la fraction surnageante par décantation. La phase aqueuse ainsi éventuellement séparée sert, après dilution, de matière première à la RP-HPLC en milieu acide décrite ci-après qui fait partie du procédé de l'invention. La dilution de la phase aqueuse est réalisée en milieu acide ayant un pH de 1 à 4, de préférence de 2 à 3. On chromatographie la phase aqueuse diluée sur une colonne à phase inversée en utilisant des supports disponibles dans le commerce tels que des silices greffées C3, C4, C8 ou C18 ayant des pores de taille convenable pour l'utilisation avec des protéines, par exemple d'un diamètre d'au moins 150 A¤ . L'élution de l'IL₂ comprend l'emploi d'un gradient de concentration croissante d'un alcool inférieur miscible à l'eau et contenant un acide organique.

Le procédé de l'invention est notamment caractérisé en ce que le stade de refroidissement éventuel est effectué dans une solution aqueuse d'acétonitrile contenant environ 0,1 % d'acide trifluoroacétique, en ce que la dilution est effectuée à l'aide d'une solution aqueuse d'un acide organique tel que l'acide citrique et en ce que ladite IL₂ est éluée dans la deuxième chromatographie, à l'aide d'une solution comprenant de l'isopropanol, de l'eau et un acide organique tel que l'acide citrique. La solution aqueuse d'acétonitrile contenant environ 0,1 % de TFA, qui est le mélange préféré soumis au stade éventuel de refroidissement que concerne l'invention, correspond à la fraction principale éluée de la première chromatographie. Très préférentiellement, la dilution de la phase aqueuse est effectuée immédiatement, soit après l'éventuelle séparation, soit après la première chromatographie. Cette dilution est réalisée par addition, de préférence, d'au moins 2 volumes d'eau contenant 0,5 à 2% d'un acide organique tel que l'acide formique, acétique, propionique, trifluoroacétique ou citrique, plus préférentiellement par addition de environ 2 volumes d'eau contenant 0,5% d'acide citrique. La deuxième chromatographie, qui fait partie du procédé revendiqué de l'invention, consiste à soumettre la phase aqueuse éventuellement séparée après le stade de refroidissement puis diluée, à une RP-HPLC utilisant des supports disponibles dans le commerce tels que des silices greffées C3, C4, C8 ou C18 ayant des diamètres de pores d'au moins 150 A¤ . Le support préféré est une silice greffée C4 VYDAC 300 A¤ qui est un gel de silice qui a des groupements butyles greffés de façon covalente, dont les pores ont un diamètre de 300 A¤ et dont les particules ont une taille moyenne de 15 à 20 microns. L'élution de l'IL₂ concernée par le procédé de l'invention est réalisée à l'aide d'un système de gradient comprenant un alcool tel que le propanol ou l'isopropanol et un acide organique tel que l'acide formique, l'acide acétique, l'acide propionique, l'acide trifluoracétique ou l'acide citrique. Le mélange préféré comprend de l'isopropanol, de l'eau et de l'acide citrique de préférence 0,5 à 2%, très préférentiellement 0,5%, et permet, à l'aide d'un système de gradient de concentration croissante d'isopropanol, l'élution d'une fraction mineure à environ 48 % d'isopropanol puis d'une fraction majeure à environ 59 % d'isopropanol, cette dernière comprenant l'IL₂ humaine recombinante non glycosylée active sous forme réduite.

L'invention a également pour objet l'IL₂ humaine recombinante non glycosylée sous forme réduite susceptible d'être obtenue par le procédé décrit ci-dessus.

La dite fraction obtenue peut être conservée de 0¤ C à -20¤ C environ. Elle est stable dans ces conditions. On peut également éliminer l'isopropanol par distillation azeotropique sous vide. La solution obtenue peut être conservée à + 4¤ C ou bien on isole directement l'IL₂ de la présente invention par lyophilisation.

L'invention concerne également une variante du prodécé dans lequel on ne soumet pas la fraction principale au stade de refroidissement et de séparation de la phase aqueuse.

La fraction principale diluée, obtenue selon la variante revendiquée du procédé de l'invention, peut être éventuellement conservée à +4¤ C. Elle est stable et constitue la matière première pour la deuxième chromatographie qui fait partie du procédé revendiqué de l'invention.

Lorsque l'on dose les groupes thiols, l'IL₂ obtenue selon l'invention présente 3 groupements sulfhydryles libres et manifeste une activité spécifique de 0.7 à 1.3 x 10⁷ u/mg dans le test de prolifération des lignées CTLL-2 c'est à dire comparable à celle de l'IL₂ native. Cette activité justifie l'un des aspects de l'invention qui concerne l'IL₂ humaine recombinante non glycosylée sous forme réduite telle que décrite ci-dessus pour son utilisation comme médicament de la même manière que l'IL₂ native, dans une variété d'indications faisant usage de son activité immunomodulatrice ainsi que de son activité antitumorale qui ont été décrites par exemple par Fletcher, M et al. Lymphokine Research 6 (1987) 47-57 et qui comprennent par exemple, la prolifération des lymphocytes T, l'induction de la cytotoxicité des cellules NK (natural killer) et des cellules LAK (lymphokine activated killer), la restauration de l'immunité cellulaire, un effet protecteur contre l'infection dans les cas de déficience immunitaire ou un effet adjuvant vis à vis de vaccins. L'administration peut être directe ou bien être une administration associée avec une méthode de l'immunothérapie adoptive qui a été décrite par Rosenberg, SA et al. dans N. Engl. J. Med. (1985) 313, 1485-1492.

Comme l'IL₂ native, l'IL₂ de l'invention peut être utilisée seule ou en association avec d'autres agents immunomodulateurs tels que l'interféron alpha, l'interféron gamma, et ou d'autres agents thérapeutiques.

L'invention a enfin pour objet une composition pharmaceutique qui renferme comme principe actif l'IL₂ humaine recombinante non glycosylée sous forme réduite définie précédemment. La composition peut être solide ou liquide. L'IL₂ de la présente invention peut être formulée selon les méthodes connues pour préparer une composition pharmaceutique utile dans laquelle l'IL₂ est combinée à un véhicule support pharmaceutiquement acceptable. Cette composition contient une quantité efficace de l'IL₂ avec une quantité appropriée de véhicule convenant à l'administration chez l'homme. La formulation peut être obtenue à partir de la solution aqueuse acide de l'IL₂ de l'invention après élimination de l'isopropanol par distillation azéotropique sous vide à laquelle on ajoute un agent de charge soluble dans l'eau et que l'on lyophilise. Par agent de charge, on entend une substance soluble dans l'eau qui ne modifie pas le pH initial lorsque le mélange est reconstitué. Des exemples des agents de charge qui peuvent être ajoutés sont des sucres tels que le glucose, le ribose, le saccharose, le maltose, le tréhalose, ou des sucres réduits tels que le mannitol. Le mannitol est préféré. On ajoute le mannitol à la concentration de 10 à 50 mg/ml, de préférence de l'ordre de 50 mg/ml, lorsque la concentration de l'IL₂ de la présente invention est comprise entre 0.05 et 1 mg/ml, selon la dose à administrer. La solution que l'on a filtrée stérilement en absence d'oxygène est répartie en flacons-dose et on lyophilise. Le mélange lyophilisé peut être reconstitué par injection, dans le flacon, d'eau distillée convenant à l'injection parentérale.

La composition de la présente invention peut être administrée par voie intraveineuse en bolus ou en perfusion continue, par voie intramusculaire, intrapéritonéale, intrapleurale ou sous cutanée. La dose efficace de l'IL₂ de l'invention, qui dépend de la voie d'administration et du malade traité, est généralement comprise entre 1 x 10⁶ U/M²/24 h et 40 x 10⁶ U/M²/24 h, de préférence de l'ordre de 20 x 10⁶ U/M²/24 h chez l'adulte ou l'enfant. La dose journalière dépend aussi de la durée de l'administration et il n'y a pas lieu de se limiter aux doses ci-dessus.

Lorsque la composition pharmaceutique est utile pour une administration en perfusion, elle est plus particulièrement caractérisée en ce qu'elle renferme de l'IL₂ humaine recombinante non glycosylée sous forme réduite, de l'eau et un acide organique tel que l'acide citrique. Une telle composition peut être obtenue à partir de la composition pharmaceutique lyophilisée de l'invention que l'on dissout en présence d'un véhicule approprié qui contribue à la stabilité du principe actif pendant la durée de la perfusion, par exemple du glucose. Les conditions préférées consistent à diluer le mélange lyophilisé reconstitué par injection d'eau distillée, en l'introduisant dans une poche à perfusion contenant un volume de glucose, à la concentration de 50 mg/ml, déterminé comme convenable pour la dose à administrer.

Toutes les publications qui sont citées sont incorporées, par référence, dans le texte de la présente demande.

Les figures ci-annexées illustrent certains aspects de l'invention :
La figure 1a. est un chromatogramme de RP-HPLC analytique de l'extrait brut en guanidine 8M de l'exemple 1.
La figure 1b. est un chromatogramme de RP-HPLC analytique des IL₂ standard réduite et oxydée.
La figure 2. est un chromatogramme de RP-HPLC analytique de la "resolubilisation" de l'exemple 1.
La figure 3. est un chromatogramme de RP-HPLC de la "fraction principale" après le stade de refroidissement de l'exemple 1.
La figure 4. est un chromatogramme de RP-HPLC analytique de la fraction "59" de l'exemple 1.
La figure 5. est le protocole de purification de l'exemple 1.
La figure 6. est le gel de l'électrophorèse SDS-PAGE de l'exemple 2.
Les figures 7a et 7b sont les cartes peptidiques de l'exemple 2.
Les figures 8a et 8b sont les courbes de DC de l'exemple 2.
La figure 9 est la courbe d'effet mitogène sur les lymphocytes humains de l'exemple 3.
La figure 10 est la courbe de toxicité obtenue vis à vis des lignées K 562 et DAUDI.

Les exemples suivants illustrent l'invention sans la limiter.

### Exemple 1 : purification de la r-hIL₂ réduite biologiquement active à partir de granules.

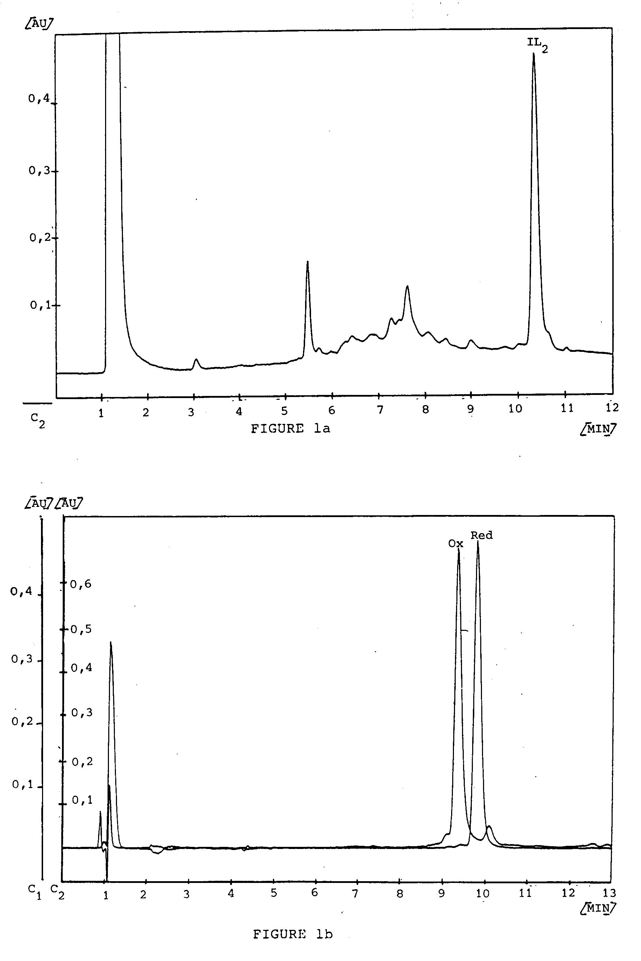

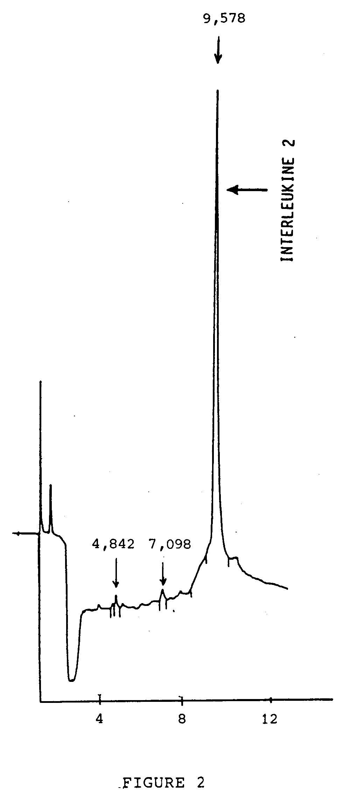

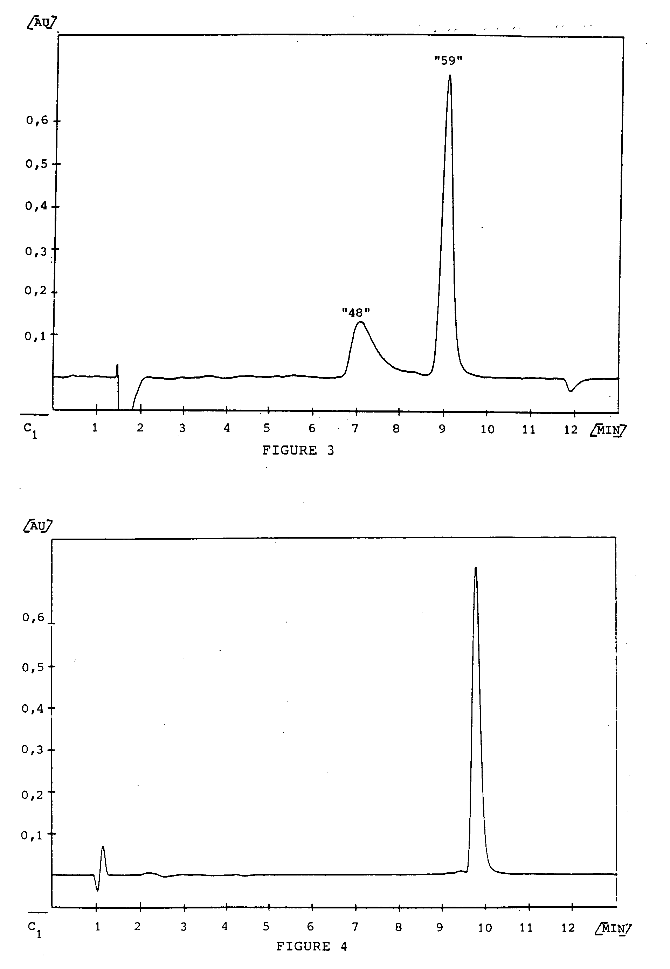

Les granules sont obtenus par centrifugation de cultures d'une souche d'E.coli transformée à l'aide d'un plasmide comprenant la séquence codant pour l'IL₂ native et capable d'accumuler l'IL₂ sous cette forme à l'intérieur des cellules, comme décrit par exemple par Sato,T et al. J. Biochem (1987) 101 525-534. Les cellules ainsi obtenues d'un fermenteur de 10 litres sont soumises à un éclatement dans un homogénéisateur Manton Gaulin. A partir des débris cellulaires isolés et lavés (90-170 g en poids humide) l'IL₂ est solubilisée dans 2.5 volumes d'un tampon Tris, HCL 20 mM pH 8 contenant du chlorhydrate de guanidine (Gu,HCl) 8M et du Dithiothréitol (DTT) 100 mM. La quantité d'IL₂ solubilisée (1.5 à 2.5 g) est estimée par RP-HPLC analytique sur une colonne C4 VYDAC (0.46 X 15 cm) 300 A¤ , 5 microns, au débit de 2 ml/mn, avec un gradient linéaire d'acétonitrile (30 à 70 % sur 10 minutes) contenant 0.1 % de TFA, une détection spectrophotométrique à 280 nm ou 210 nm, dont on évalue la surface du pic à 280 nm après calibration avec un standard d'IL₂ (fig. 1a et fig. 1b). L'IL₂ est ensuite précipitée par abaissement à 2M de la concentration en Gu, HCl, en présence de DTT. Après lavage du précipité à l'aide d'une solution aqueuse de TFA à 0.1 % jusqu'à obtention d'un pH du surnageant inférieur à 5.0, l'IL₂ est solubilisée dans une solution aqueuse à 20 % d'acétonitrile et 0.1 % de TFA. La "resolubilisation" obtenue, qui renferme selon la RP-HPLC analytique (fig. 2) une teneur en IL₂ réduite supérieure à 85 %, a une activité biologique inférieure à 0.01 x 10⁷ U/mg d'IL₂ réduite et une teneur en groupements sulfhydryles de 2.85 SH/mole d'IL₂ réduite. Une fraction de la solution obtenue, correspondant à environ 200 mg d'IL₂ estimée par RP-HPLC analytique, est diluée de façon à ajuster la concentration en acétonitrile inférieure à 10 % dans du TFA à 0.1 %, puis appliquée à une colonne C4 VYDAC (5.7 x 30 cm). L'IL₂ est éluée au débit de 100 ml/mn, à l'aide d'un gradient linéaire d'acétonitrile (30 à 80 % sur 40 minutes) contenant 0.1 % de TFA, à une concentration d'environ 60 % en acétonitrile dans un pic majeur détecté par spectrophotométrie à 280 nm et analysé par RP-HPLC. La "fraction principale" recueillie qui renferme l'IL₂ réduite est ensuite soumise à un stade de refroidissement lent, de la température ambiante à -20¤ C ± 1¤ , qui permet d'éliminer par décantation une phase organique supérieure riche en acétonitrile. La phase inférieure, riche en eau et contenant l'IL₂, peut être conservée à l'état congelé. Après décongélation, la solution obtenue est, après dilution par 2 volumes de solution aqueuse d'acide citrique à 0.5 %, appliquée à une colonne C4 VYDAC (5.7 x 30 cm) que l'on développe avec un gradient linéaire d'isopropanol (20 à 70 % sur 40 minutes) contenant 0.5 % d'acide citrique, au débit de 50 ml/mn. L'effluent, suivi par spectrophotométrie à 280 nm, montre l'élution successive d'un pic mineur à une concentration d'environ 48 % en isopropanol (fraction "48") et d'un pic majeur à une concentration d'environ 59 % en isopropanol (fraction "59") (fig. 3). La fraction "59" est recueillie. Elle est stable à la conservation à 0¤ C pendant au moins 24 h, à l'abri de l'air.

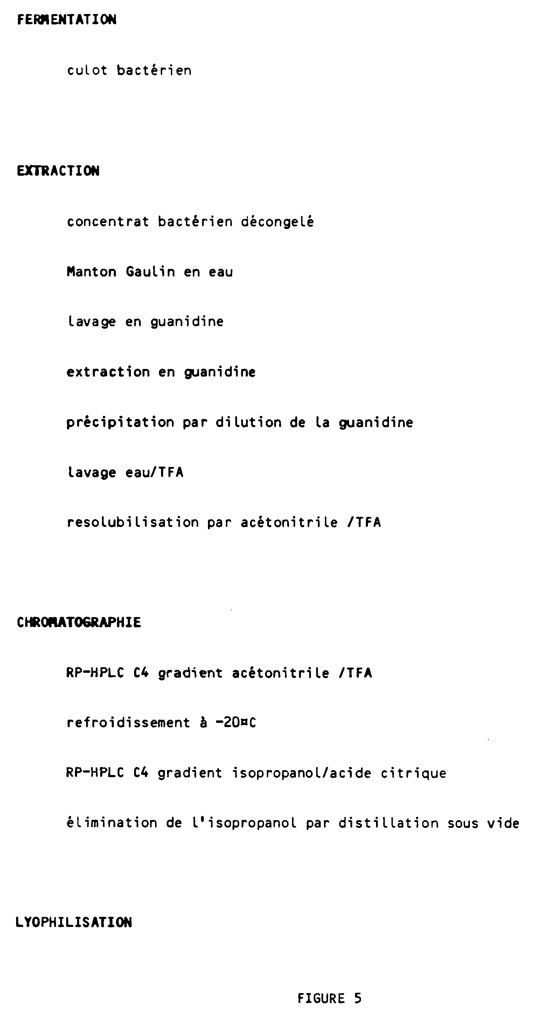

Après élimination de l'isopropanol par distillation azéotropique sous vide, la fraction "59" est analysée en RP-HPLC analytique et donne un pic homogène (fig. 4) élué à environ 60 % d'acétonitrile alors que l'IL₂ oxydée de référence est éluée à environ 57 % d'acétonitrile (fig. 1b). La fraction "59" qui, après élimination de l'isopropanol, a une concentration en IL₂ supérieure à 1 mg/ml et un pH de 3 ± 0.5, peut être conservée à +4¤ C, à l'abri de l'air, pendant au moins une semaine ou être immédiatement lyophilisée ou formulée pour l'obtention d'une composition pharmaceutique. La fraction "59" lyophilisée est dosée en activité biologique, selon le test in vitro de prolifération de cellules CTLL-2, et a une activité spécifique de 1.3 ± 0.5 x 10⁷ U/mg comparable à celle de l'IL₂ native.

La teneur en groupements sulfhydryles libres de la fraction "59" lyophilisée, déterminée par la méthode colorimétrique à la dithiodipyridine, est de 2.94 SH/mole comparativement à 0.76 SH/mole pour l'IL₂ oxydée de référence.

150 à 300 mg titrés par RP-HPLC analytique de r-hIL₂ réduite, contenant 3 groupements SH, biologiquement active, homogène en RP-HPLC, sont obtenus dans la fraction "59" à partir d'un fermenteur de 10 litres (selon le schéma de protocole fig. 5).

### Exemple 2 : purification de la r-Il₂ réduite biologiquement active.

On opère comme dans l'exemple 1 sauf que l'on ne soumet pas la "fraction principale" au stade de refroidissement puis de séparation par décantation mais la dilue immédiatement par 2 volumes de solution aqueuse d'acide citrique à 0,5%. La fraction "59" est recueillie et traitée comme dans l'exemple 1.

### Exemple 3 : caractérisation physicochimique de la r-hIL₂ réduite biologiquement active.

La r-hIL₂ réduite obtenue selon l'invention dans la fraction "59" de l'exemple 1 est examinée pour les propriétés suivantes :

### 1) Homogénéité

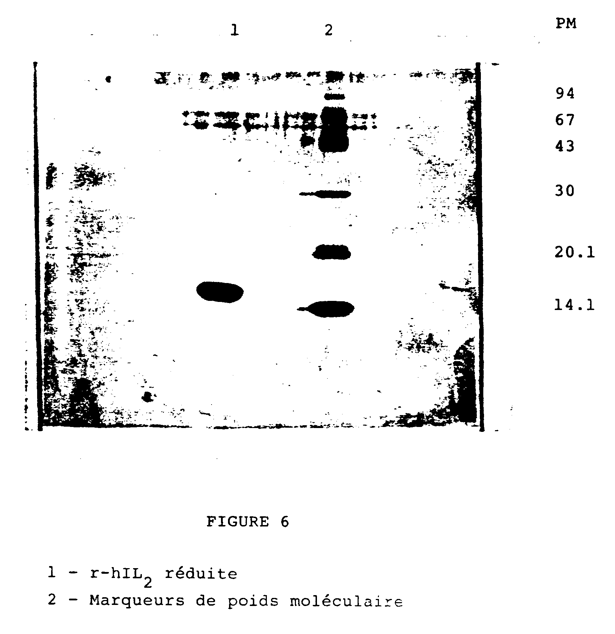

L'électrophorèse SDS PAGE est effectuée sur un gel à deux phases (gel de concentration et gel de migration respectivement à 5 % et 15 % en acrylamide) contenant 10 % de SDS. L'échantillon est chauffé au préalable pendant 2 mn à 100¤ C dans un tampon à 3 % de SDS et 5 % de mercaptoéthanol. La migration avec un tampon à 1 % de SDS est suivie d'une coloration à l'argent qui révèle une seule bande correspondant à une pureté supérieure à 99 % pour un dépôt de 2 ug (fig. 6).

### 2) Poids moléculaire par électrophorèse

En milieu réducteur, on détermine un PM apparent de environ 15 Kd, en accord avec le PM calculé de 15420 (fig. 6).

### 3) Composition en acides aminés

Un échantillon, contenant 25 ug de la r-hIL₂ réduite de l'invention dans 0.5 ml d'eau, est placé dans un tube à hydrolyse en verre auquel sont ajoutés 0.5 ml d'acide chlorhydrique concentré à 31.7 % de TFA et 4.8 % d'acide thioglycolique. Le tube est scellé sous vide, puis l'hydrolyse est effectuée à 155¤ C pendant 40 mn. L'hydrolysat est ensuite évaporé à sec sous pression réduite. Le résidu est dissous dans 0.7 ml de tampon citrate pH=3, puis est soumis à l'analyse en acides aminés sur une colonne Interaction AA 511 (0.46 x 15 cm) à l'aide d'un gradient de pH (3 à 5) et de chlorure de sodium (0 à 70 g/l) dans le tampon citrate, à 60¤ C, au débit de 0.5 ml/mn et une détection par fluorescence, après dérivation à l'orthophtalaldéhyde à la sortie de la colonne. Les résultats sont indiqués dans le tableau 1. Les valeurs sont la moyenne obtenue pour 2 hydrolyses répétées et 2 chromatographies respectivement répétées. La composition est en accord avec celle de l'IL₂ native ayant une méthionine N terminale supplémentaire.

**TABLEAU 1**

| **INTERLEUKINE 2** | | |
|---|---|---|
| Acides Aminés | Nombre théorique | Nombre trouvé |
| GLN + GLU | 18 | 17,56 |
| ASN + ASP | 12 | 12,61 |
| THR | 13 | 12,09 |
| SER | 8 | 7,05 |
| PRO | 5 | ND* |
| GLY | 2 | 2,59 |
| ALA | 5 | 5,50 |
| VAL | 4 | 4,34 |
| MET | 5 | 5,59 |
| ILE | 9 | 9,15 |
| LEU | 22 | 19,96 |
| TYR | 3 | 2,60 |
| PHE | 6 | 5,90 |
| TRP | 1 | NC** |
| LYS | 11 | 10,19 |
| HIS | 3 | 3,48 |
| ARG | 4 | 5,29 |
| CYS | 3 | NC** |

| | | |
|---|---|---|
| * ND = non détecté | | |
| ** NC = non calculé | | |

### 4) Séquence en aminoacide N terminale

La séquence N terminale est identifiée par microséquençage en utilisant la méthode automatique de dégradation d'Edman : l'analyse de 15 ug de r-hIL₂ réduite, biologiquement active selon l'invention, sur un microséquenceur phase gaz Applied Biosystems 470A couplé à HPLC 120A, permet d'identifier les PTH acides aminés suivants :

| Etapes | PTH AA | Etapes | PTH AA |
|---|---|---|---|
| 1 | Met | 11 | Thr |
| | Ala | | |
| 2 | Ala | 12 | Gln |
| | Pro | | |
| 3 | Pro | 13 | Leu |
| | Thr | | |
| 4 | Thr | 14 | Gln |
| | Ser | | |
| 5 | Ser | 15 | Leu |
| 6 | Ser | 16 | Glu |
| 7 | Ser | 17 | His |
| 8 | Thr | 18 | Leu |
| 9 | Lys | 19 | Leu |
| 10 | Lys | 20 | Leu |

La séquence des 20 résidus N-terminaux est en accord avec l'ordre d'enchainement théorique de l'IL₂ native. On observe environ 10 % d'IL₂ sans méthionine.

### 5) Carte peptidique au bromure de cyanogène

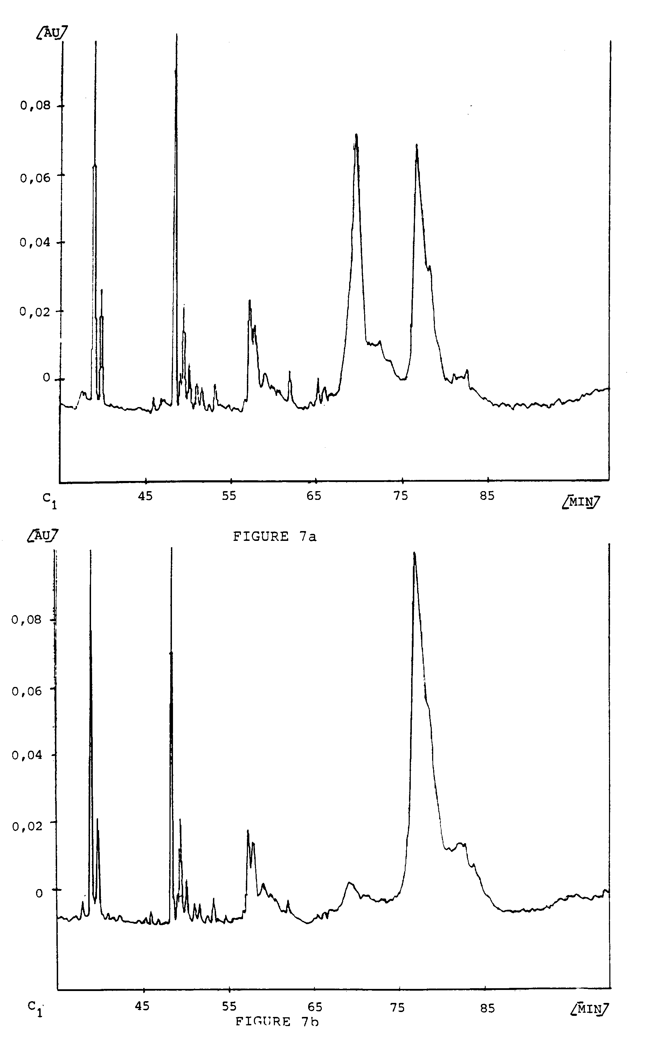

700 ug d'IL₂ réduite selon l'invention (environ 53 nmoles) sont dissous dans 4 ml d'acide formique à 70 % et additionnés de 5.6 mg de bromure de cyanogène. La solution est agitée pendant une nuit à température ambiante, diluée à l'eau puis lyophilisée. Le mélange réactionnel est analysé par RP-HPLC sur une colonne uBondapack C18 RP (0.46 x 20 cm), à l'aide d'un gradient de concentration d'acétonitrile variant de 0 à 70 % contenant 0.1 % de TFA, au débit de 1 ml/mn, à température ambiante et avec une détection spectrophotométrique à 220 nm. La carte peptidique de la r-hIL₂ réduite (fig. 7a) montre une fragmentation différente de celle de l'IL₂ oxydée de référence (fig. 7b).

### 6) Dichroisme circulaire

Les spectres de dichroisme circulaire (DC) sont déterminés à température ambiante sur un spectrographe Jobin Yvon Mark V. L'échantillon de r-hIL₂ réduite, lyophilisé après RP-HPLC réalisée avec le gradient linéaire d'isopropanol dont on a remplacé l'acide citrique 0.5 % selon l'exemple 1, par l'acide formique 0.1 %, puis distillation de l'isopropanol et lyophilisation, est repris à la concentration de 1 mg/ml dans l'acide acétique. Les cuves utilisées sont de 0.01 cm et 0.5 cm respectivement pour la région peptidique (185-250 nm) et la région aromatique (260-320 nm). Le spectre du solvant est soustrait du spectre de l'IL₂_{,} pour chaque échantillon. Les résultats sont donnés en ellipticité 0 (poids moyen par résidu d'IL₂=116).

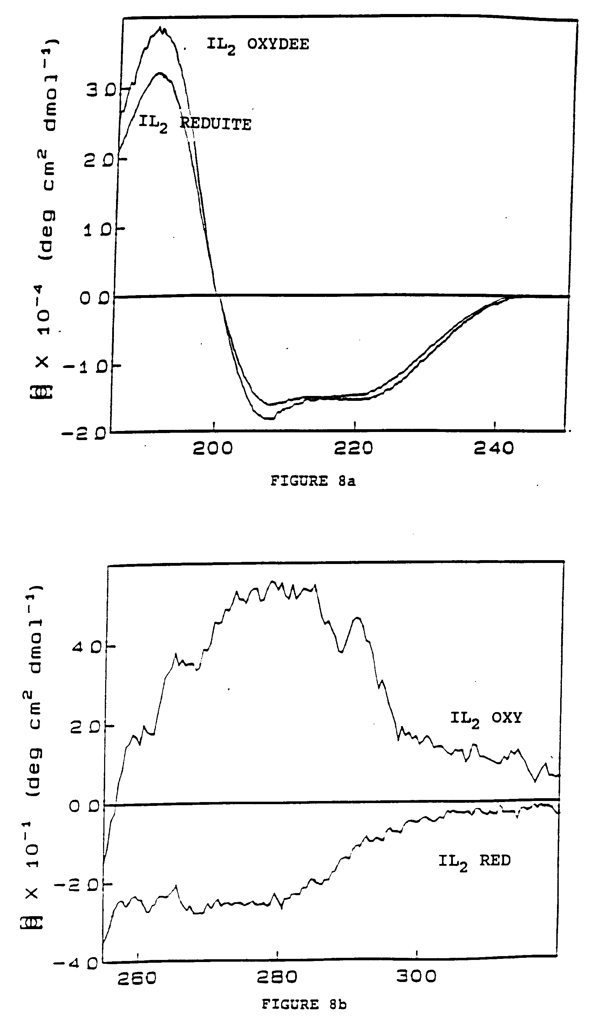

La figure 8a montre le spectre de DC dans l'UV lointain : la r-hIL₂ réduite biologiquement active, selon l'invention, a un spectre qui indique la présence d'une structure secondaire ordonnée. La détermination des % d'hélice alpha montre peu de différence significative avec l'IL₂oxydée de référence (% hélice alpha # 50 %).

La figure 8b montre le spectre de DC dans l'UV proche : l'IL₂ oxydée de référence à un DC significatif qui indique un environnement asymétrique pour les résidus aromatiques, tandis que la r-hIL₂ réduite de l'invention a un DC peu significatif mais différent.

### Exemple 4 : activité biologique.

L'efficacité biologique de la r-hIL₂ réduite de l'invention est évaluée dans des expériences in vitro ou ex vivo.

### 1) Activité in vitro sur cellules humaines

### a. test de transformation lymphoblastique

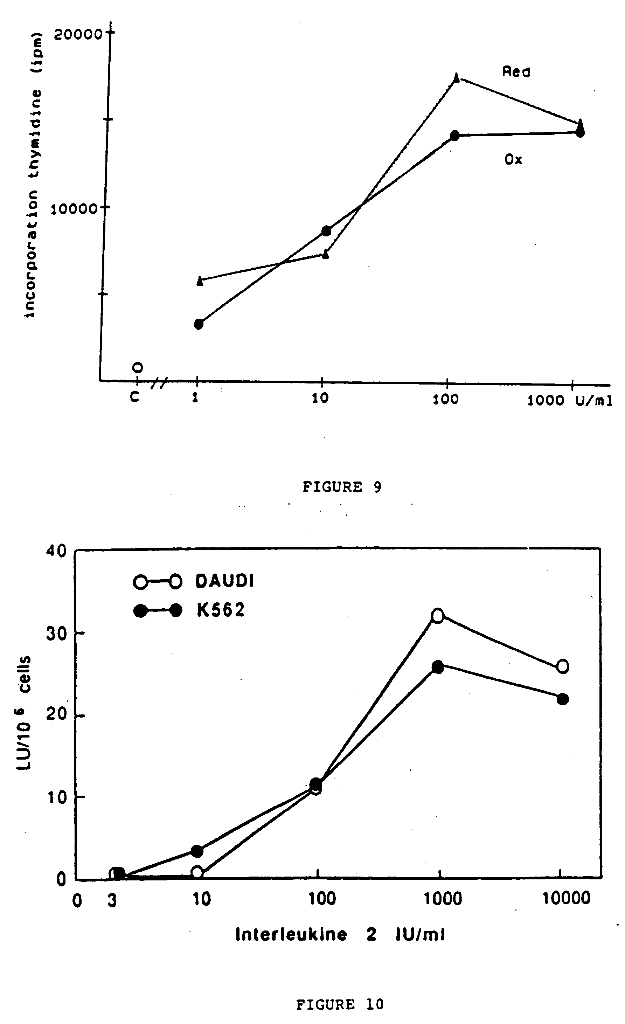

Outre l'activité proliférative sur lignées cellulaires de souris telles que CTLL-2 qui permet le dosage biologique de l'IL₂, la r-hIL₂ réduite de l'invention manifeste un effet mitogène, dépendant de la dose, comparable à celui montré par l'IL₂ oxydée de référence sur les lymphocytes humains circulants normaux, montré par mesure de l'incorporation de thymidine tritiée dans l'ADN (fig. 9).

### b. induction de la cytotoxicité des cellules mononuclées

L'étude est réalisée avec des cellules mononuclées circulantes humaines incubées en présence d'IL₂ et dont on détermine l'effet cytotoxique vis à vis de cellules cibles tumorales, respectivement la lignée erythroleucémique K 562 (sensible aux cellules NK) et la lignée DAUDI dérivée d'un lymphome B (résistante aux cellules NK), par mesure du relargage de Cr 51 en 4 heures. Les résultats, exprimés en unités lytiques par 10⁶ cellules (UL/10⁶), montre que la r-hIL₂ réduite de l'invention manifeste une capacité, dépendante de la dose, respectivement à augmenter l'activité NK ou à induire la cytotoxicité des lymphocytes T Vis à vis de cibles tumorales, de façon comparable à celle connue pour l'IL₂ native (fig. 10).

### 2) Activité ex vivo sur la souris (stimulation des macrophages péritonéaux)

Chez la souris normale Balb/c et MRL-+/+ l'injection intrapéritonéale successive d'Interféron gamma recombinant de rat à doses suboptimales (100 à 3000 U), puis 24 heures plus tard, de la r-hIL₂ réduite de l'invention, déclenche les mécanismes oxydatifs des cellules phagocytaires, évalués par mesure de la chimiluminescence, en présence d'ester de phorbol (PMA), des cellules prélevées de la cavité péritonéale des souris sacrifiées 24 h après l'injection d'IL₂. L'effet, dépendant de la dose, est comparable à celui observé avec l'IL₂ oxydée de référence.

### Résultats :

| Dose Injectée (ng par souris) | Chimiluminescence (CPM x 10⁴) Après injection de r-hIL₂ | |
|---|---|---|
| | Réduite | Oxydée |
| 0 | 5 ± 2 | 4 ± 3 |
| 1 | 7 ± 3 | 5 ± 1 |
| 3 | 90 ± 5 | 71 ± 5 |
| 10 | 130 ± 10 | 140 ± 70 |
| 30 | 350 ± 50 | 400 ± 70 |
| 100 | 710 ± 20 | 695 ± 25 |
| 300 | 375 ± 25 | 350 ± 20 |

### Exemple 5 : composition pharmaceutique pour infection.

La solution aqueuse de r-hIL₂ réduite correspondant à la fraction "59" de l'invention dont on a éliminé l'isopropanol par distillation azéotropique sous vide est extemporanément diluée à l'aide d'une solution aqueuse de mannitol dégazée et saturée en azote, à raison de 100 µg d'IL₂ réduite/ml et 50 mg/ml de mannitol. Après filtration sur une membrane de 0.22 µ, répartition stérile de 1 ml dans des flacons et lyophilisation, les flaconsdose sont bouchés sous atmosphère d'azote et conservés à une température de +4¤ C avant usage.

### Exemple 6 : Composition pharmaceutique par perfusion continue.

La solution aqueuse de r-hIL₂ réduite correspondant à la fraction "59" de l'invention dont on a éliminé l'isopropanol par distillation azéotropique sous vide est extemporanément diluée à l'aide d'une solution aqueuse de mannitol dégazée et saturée en azote, à raison de 500 µg d'IL₂ réduite/ml et 50 mg/ml de mannitol. Après filtration sur une membrane de 0.22 µ, répartition stérile de 1ml dans des flacons de lyophilisation, les flacons-dose sont bouchés sous atmosphère d'azote et conservés à une température de 4¤ C avant usage. Le contenu de chaque flacon est ensuite dissous par injection de 1ml d'eau distillée stérile. Les solutions correspondant à 7 flacons-dose (environ 35.16⁶ unités) sont introduites dans un conteneur Viaflex ^{R} contenant 500 ml de soluté pour perfusion Travenol ^{R} Glucose à 5%.

## Revendications (Revendications pour l'(les) Etat(s) contractant(s) suivant(s): AT, BE, CH, DE, FR, GB, IT, LI, LU, NL, SE)

1. Interleukine 2 humaine recombinante non glycosylée ayant d'une part la séquence en acides aminés suivante : 
X-






.
 ainsi que les allèles ou dérivés de cette séquence dans laquelle X représente une méthionine ou un atome d'hydrogène et dont les trois cystéines en position 58, 105 et 125 sont sous la forme réduite, d'autre part manifestant une activité biologique comparable à celle de l'IL₂ oxydée ayant la même séquence et comportant un pont disulfure en position 58-105.

2. Interleukine 2 humaine recombinante non glycosylée ayant d'une part la séquence en acides aminés suivante : 
X-







 ainsi que les allèles ou dérivés de cette séquence dans laquelle X représente une mèthionine ou un atome d'hydrogène et dont les trois cystéines en position 58, 105 et 125 sont sous la forme réduite, d'autre part manifestant une activité biologique d'au moins 0.5 x 10⁷U/mg.

3. Interleukine 2 humaine recombinante non glycosylée ayant la séquence en acides aminés suivante : 
) X-[?]a Pro Thr Ser Ser Ser Thr Lys Lys Thr Gln Leu Gln Leu Glu His Leu Leu
Le[?] Asp Leu Gln Met Ile Leu Asn Gly Ile Asn Asn Tyr Lys Asn Pro Lys Leu Thr
 
5 Arg Met Leu Thr Phe Lys Phe Tyr Met Pro Lys Lys Ala Thr Glu Leu Lys His Leu

Gln Cys Leu Glu Glu Glu Leu Lys Pro Leu Glu Glu Val Leu Asn Leu Ala Gln Ser

Lys Asn Phe His Leu Arg Pro Arg Asp Leu Ile Ser Asn Ile Asn Val Ile Val Leu

0 Glu Leu Lys Gly Ser Glu Thr Thr Phe Met Cys Glu Tyr Ala Asp Glu Thr Ala Thr

Ile Val Glu Phe Leu Asn Arg Trp Ile Thr Phe Cys Gln Ser Ile Ile Ser Thr Leu

Thr
 dans laquelle X représente une méthionine ou un atome d'hydrogène et dont les trois cystéines en position 58, 105 et 125 sont sous la forme réduite et manifestant une activité biologique comparable à celle de l'IL₂ humaine native.

4. Procédé de préparation de l'IL₂ humaine recombinante non glycosylée réduite, telle que définie à l'une des revendications 1 à 3, comprenant d'abord l'extraction de l'IL₂ accumulée sous forme de granules dans un microorganisme transformé par solubilisation en milieu réducteur à l'aide d'un agent chaotrope, puis la purification par précipitation suivie d'une chromatographie liquide à haute performance en phase inversée avec a éluant acide, et caractérisé en ce que :
a) le cas échéant l'on soumet la fraction principale éluée de la dite chromatographie à un stade de refroidissement à une température de l'ordre de -20◊C, puis à une séparation de la phase aqueuse que
b) l'on dilue en milieu acide, puis chromatographie sur une autre colonne de chromatographie liquide à haute performance en phase inversée en milieu acide et isole ladite IL₂.

5. Procédé selon la revendication 4, caractérisé en ce que le stade de refroidissement est effectué dans une solution aqueuse d'acétonitrile contenant environ 0,1% d'acide trifluoroacétique, en ce que la dilution est effectuée à l'aide d'une solution aqueuse d'un acide organique tel que l'acide citrique et en ce que ladite IL₂ est éluée dans la deuxième chromatographie, à l'aide d'une solution comprenant de l'isopropanol, de l'eau et un acide organique tel que l'acide citrique.

6. Procédé selon la revendication 4 ou 5, caractérisé en ce que l'on ne soumet pas la fraction principale au stade de refroidissement et de séparation de la phase aqueuse.

7. IL₂ humaine recombinante non glycosylée sous forme réduite susceptible d'être obtenue par le procédé selon l'une des revendications 4 à 6.

8. IL₂ humaine recombinante non glycosylée sous forme réduite, telle que définie selon l'une des revendications 1 à 3 ou 7, pour son utilisation comme médicament.

9. Composition pharmaceutique, caractérisée en ce qu'elle renferme comme principe actif l'IL₂ humaine recombinante non glycosylée sous forme réduite, telle que définie selon la revendication 8.

10. Composition pharmaceutique, caractérisée en ce qu'elle renferme de l'IL₂ humaine recombinante non glycosylée sous forme réduite telle que définie selon la revendication 8, de l'eau et un acide organique tel que l'acide citrique.

## Revendications (Revendications pour l'(les) Etat(s) contractant(s) suivant(s): ES, GR)

1. Procédé de préparation de l'Interleukine 2 humaine recombinante non glycosylée ayant d'une part la séquence en acides aminés suivante : 
X-






.
 ainsi que les allèles ou dérivés de cette séquence dans laquelle X représente une mèthionine ou un atome d'hydrogène et dont les trois cystèines en position 58, 105 et 125 sont sous la forme réduite, d'autre part manifestant une activité biologique comparable à celle de l'IL₂ oxydée ayant la même séquence et comportant un pont disulfure en position 58-105, comprenant d'abord l'extraction de l'IL₂ accumulée sous forme de granules dans un microorganisme transformé par solubilisation en milieu réducteur à l'aide d'un agent chaotrope, puis la purification par précipitation suivie d'une chromatographie liquide à haute performance en phase inversée avec un éluant acide, et caractérisé en ce que :
a) le cas échéant l'on soumet la fraction principale éluée de la dite chromatographie à un stade de refroidissement à une température de l'ordre de -20◊C, puis à une séparation de la phase aqueuse que
b) l'on dilue en milieu acide, puis chromatographie sur une autre colonne de chromatographie liquide à haute performance en phase inversée en milieu acide et isole ladite IL₂.

2. Procédé selon la revendication 1, caractérisé en ce que le stade de refroidissement est effectué dans une solution aqueuse d'acétonitrile contenant environ 0,1% d'acide trifluoroacétique, en ce que la dilution est effectuée à l'aide d'une solution aqueuse d'un acide organique tel que l'acide citrique et en ce que ladite IL₂ est éluée dans la deuxième chromatographie, à l'aide d'une solution comprenant de l'isopropanol, de l'eau et un acide organique tel que l'acide citrique.

3. Procédé selon la revendication 1 ou 2, caractérisé en ce que l'on ne soumet pas la fraction principale au stade de refroidissement et de séparation de la phase aqueuse.

4. Procédé selon l'une quelque des revendications 1 à 3, caractérisé en ce que l'on prépare un produit manifestant une activité biologique d'au moins 0.5 x 10⁷U/mg.

5. Procédé selon l'une quelconque des revendications 1 à 4, caractérisé en ce que l'on prépare un produit manifestant une activité biologique comparable à celle de l'IL₂ humaine native.

## Claims (Claims for the following Contracting State(s): AT, BE, CH, DE, FR, GB, IT, LI, LU, NL, SE)

1. Non-glycosylated recombinant human interleukin 2 having on the one hand the following amino acid sequence: 
X-






.
 as well as the alleles or derivatives of this sequence in which X represents a methionine or a hydrogen atom and of which the three cysteines in position 58, 105 and 125 are in reduced form, on the other hand having a biological activity comparable with that of oxidized IL₂ having the same sequence and containing a disulphide bridge in position 58-105.

2. Non-glycosylated recombinant human interleukin 2 having on the one hand the following amino acid sequence: 
X-






.
 as well as the alleles or derivatives of this sequence in which X represents a methionine or a hydrogen atom and of which the three cysteines in position 58, 105 and 125 are in reduced form, on the other hand having a biological activity of at least 0.5 x 10⁷U/mg.

3. Non-glycosylated recombinant human interleukin 2 having the following amino acid sequence: 
X-






.
 in which X represents a methionine or a hydrogen atom and of which the three cysteines in position 58, 105 and 125 are in reduced form and having a biological activity comparable with that of native human IL₂.

4. Preparation process for reduced non-glycosylated recombinant human IL₂ as defined in one of claims 1 to 3, comprising first of all the extraction of IL₂ accumulated in the form of granules in a microorganism converted by solubilization in a reducing medium using a chaotropic agent, then purification by precipitation followed by a reversed-phase high-performance liquid chromatography with an acid eluant, and characterized in that:
a) if appropriate, the main eluted fraction of the said chromatography is subjected to a cooling down stage to a temperature of the order of -20°C, then to a separation of the aqueous phase which
b) is diluted in an acid medium, then chromatographed on another reversed-phase high-performance liquid chromatography column in an acid medium and the said IL₂ is isolated.

5. Process according to claim 4, characterized in that the cooling down stage is carried out in an aqueous solution of acetonitrile containing about 0.1% of trifluoroacetic acid, and in that the dilution is carried out using an aqueous solution of an organic acid such as citric acid and in that the said IL₂ is eluted in a second chromatography, using a solution containing isopropanol, water and an organic acid such as citric acid.

6. Process according to claim 4 or 5, characterized in that the main fraction is not subjected to the cooling down stage and separation of the aqueous phase.

7. Non-glycosylated recombinant human IL₂ in reduced form which can be obtained by the process according to one of claims 4 to 6.

8. Non-glycosylated recombinant human IL₂ in reduced form, as defined according to one of claims 1 to 3 or 7, for its use as a medicament.

9. Pharmaceutical composition, characterized in that it contains as active ingredient non-glycosylated recombinant IL₂ in reduced form, as defined according to claim 8.

10. Pharmaceutical composition, characterized in that it contains non-glycosylated recombinant human IL₂ in reduced form as defined according to claim 8, water and an organic acid such as citric acid.

## Claims (Claims for the following Contracting State(s): ES, GR)

1. Preparation process for non-glycosylated recombinant human interleukin 2 having on the one hand the following amino acid sequence: 
X-






.
 as well as the alleles or derivatives of this sequence in which X represents a methionine or a hydrogen atom and of which the three cysteines in position 58, 105 and 125 are in reduced form, on the other hand having a biological activity comparable with that of oxidized IL₂ having the same sequence and containing a disulphide bridge in position 58-105, comprising first of all the extraction of IL₂ accumulated in the form of granules in a microorganism converted by solubilization in a reducing medium with a chaotropic agent, then purification by precipitation followed by a reversed-phase high-performance liquid chromatography with an acid eluant, and characterized in that:
a) if appropriate, the main eluted fraction of the said chromatography is subjected to a cooling down stage to a temperature of the order of -20°C, then to a separation of the aqueous phase which
b) is diluted in an acid medium, then chromatographed on another reversed-phase high-performance liquid chromatography column in an acid medium and the said IL₂ is isolated.

2. Process according to claim 1, characterized in that the cooling down stage is carried out in an aqueous solution of acetonitrile containing about 0.1% of trifluoroacetic acid, in that the dilution is carried out using an aqueous solution of an organic acid such as citric acid and in that the said IL₂ is eluted in the second chromatography using a solution containing isopropanol, water and an organic acid such as citric acid.

3. Process according to claim 1 or 2, characterized in that the main fraction is not subjected to the cooling down stage and separation of the aqueous phase.

4. Process according to any one of claims 1 to 3, characterized in that a product is prepared which has a biological activity of at least 0.5 x 10⁷U/mg.

5. Process according to any one of claims 1 to 4, characterized in that a product is prepared which has a biological activity comparable with that of native human IL₂.

## Patentansprüche (Patentansprüche für folgende(n) Vertragsstaat(en): AT, BE, CH, DE, FR, GB, IT, LI, LU, NL, SE)

1. Rekombinantes unglykosyliertes Humen-Interleukin-2, das einerseits folgende Aminosäurensequenz aufweist: 
X-






,
 sowie die Allele oder Derivate dieser Sequenz, in der X für ein Methionin oder ein Wasserstoffatom steht und dessen drei Cysteine in Position 58, 105 und 125 in reduzierter Form vorliegen, andererseits eine biologische Aktivität aufweist, die vergleichbar ist mit der des oxidierten IL-2 mit der gleichen Sequenz und mit einer Disulfidbrücke in Position 58-105.

2. Rekombinantes unglykosyliertes Human-Interleukin-2, das einerseits folgende Aminosäurensequenz aufweist: 
X-






,
 sowie die Allele oder Derivate dieser Sequenz, in der X für ein Methionin oder ein Wasserstoffatom steht und dessen drei Cysteine in Position 58, 105 und 125 in reduzierter Form vorliegen, das andererseite eine biologische Aktivität von wenigstens 0,5 x 10⁷ U/mg aufweist.

3. Rekombinantes unglykosyliertes Human-Interleukin -2 mit folgender Aminosäurensequenz: 
X-






,
 in der X für ein Methionin oder ein Wasserstoffatom steht und dessen drei Cysteine in Position 58, 105 und 125 in reduzierter Form vorliegen und das eine biologische Aktivität aufweist, die vergleichbar ist mit der des nativen Human-IL-2.

4. Verfahren zur Herstellung von rekombinantem unglykosyliertem reduziertem Human-IL-2. wie es in einem der Ansprüche 1 bis 3 definiert ist, ein Verfahren, das zuerst die Extraktion des IL-2 beinhaltet, das in Form von Granula in einem transformierten Mikroorganismus angereichert ist, und zwar durch Solubilisierung in reduzierendem Medium mit Hilfe eines chaotropen Mittels. dann die Reinigung durch Präzipitation, gefolgt von einer Hochleistungsflüssigchromatographie auf Umkehrphase mit einen, sauren Eluenten. und dadurch gekennzeichnet, daß man
a) vorkommendenfalls die eluierte Hauptphase der besagten Chromatographie auf eine Temperatur im Bereich von -20°C abkühlt, dann die wäßrige Phase abtrennt, die man
b) in saurem Medium verdünnt, dann über eine andere Hochleistungsflüssigchromatographiesäule in Umkehrphase in saurem Medium chromatographisch auftrennt und besagtes IL-2 isoliert.

5. Verfahren nach Anspruch 4, dadurch gekennzeichnet, daß der Schritt des Abkühlens in einer wäßrigen Acetonitrillösung durchgeführt wird, die etwa 0.1% Trifluoressigsäure enthält, dadurch daß die Verdünnung mit Hilfe einer wäßrigen Lösung einer organischen Säure wie Citronensäure durchgeführt wird, und dadurch, daß besagtes IL-2 in der zweiten Chromatographie mit Hilfe einer Lösung aus Isopropanol, Wasser und einer organischen Säure wie Citronensäure eluiert wird.

6. Verfahren nach Anspruch 4 oder 5, dadurch gekennzeichnet, daß man die Hauptfraktion nicht dein Schritt des Abkühlens und der Abtrennung der wäßrigen Phase unterzieht.

7. Rekombinantes unglykosyliertes Human- IL-2 in reduzierter Form, die nach dem Verfahren nach einer der Andprüche 4 bis 6 erhalten werden kann.

8. Rekombinantes unglykosyliertes IL-2 in reduzierter Form, wie es nach einem der Ansprüche 1 bis 3 oder 7 definiert ist, für seine Verwendung als Arzneimittel.

9. Pharmazeutische Zusammensetzung, dadurch gekennzeichnet, daß sie als Wirkstoff das rekombinante unglykosylierte Human-IL-2 in reduzierter Form enthält, wie es nach Anspruch 8 definiert ist.

10. Pharmazeutische Zusammensetzung, dadurch gekennzeichnet, daß sie das rekombinante unglykosylierte Human-IL-2 in redizierter Form, wie es nach Anspruch 8 definiert ist, Wasser und eine organische Säure wie Citronensäure enthält.

## Patentansprüche (Patentansprüche für folgende(n) Vertragsstaat(en): ES, GR)

1. Verfahren zur Herstellung von rekombinantem unglykosyliertem Human-Interleukin -2, das einerseits folgende Aminosäurensequenz aufweist: 
X-






,
 sowie die Allele oder Derivate dieser Sequenz, in der X für ein Methionin oder ein Wasserstoffatom steht und dessen drei Cysteine in Position 58, 105 und 125 in reduzierter Form vorliegen, das andererseits eine biologische Aktivität aufweist, die mit der des oxidierten IL-2 mit der gleichen Sequenz und mit einer Disulfidbrücke in Position 58-105 vergleichbar ist, ein Verfahren, das zuerst die Extraktion des IL-2 beinhaltet, das in Form von Granula in einem transformierten Mikroorganismus angereichert ist, und zwar durch Solubilisierung in reduzierendem Medium mit Hilfe eines chaotropen Mittels, dann die Reinigung durch Präzipitation, gefolgt von einer Hochleistungsflüssigchromatographie in Umkehrphase mit einem sauren Eluent, und das dadurch gekennzeichnet ist, daß man
a) vorkommendenfalls die eluierte Hauptphase der besagten Chromatographie auf eine Temperatur im Bereich von -20°C abkühlt, dann die wäßrige Phase abtrennt, die man
b) in saurem Medium verdünnt, dann über eine andere Hochleistungsflüssigchrontatographiesäule in Umkehrphase in saurem Medium chromatographisch auftrennt und besagtes IL-2 isoliert.

2. Verfahren nach Anspruch 1, dadurch gekennzeichnet, daß der Schritt des Abkühlens in einer wäßigen Acetonitrillösung durchgeführt wird, die etwa 0.1% Trifluoressigsäure enthält, dadurch daß die Verdünnung mit Hilfe einer wäßrigen Lösung einer organischen Säure wie Citronensäure durchgeführt wird und dadurch, daß besagtes IL-2 in der zweiten Chromatographie mit Hilfe einer Lösung aus Isopropanol, Wasser und einer organischen Säure wie Citronensäure eluiert wird.

3. Verfahren nach Anspruch 1 oder 2, dadurch gekennzeichnet, daß man die Hauptfraktion nicht dent Schritt des Abkühlens und der Abtrennung der wäßrigen Phase unterzieht.

4. Verfahren nach einem beliebigen der Ansprüche 1 bis 3, dadurch gekennzeichnet, daß man ein Produkt herstellt, das eine biologische Aktivität von wenigstens 0,5 x 10⁷ U/mg aufweist.

5. Verfahren nach einem beliebigen der Ansprüche 1 bis 4, dadurch gekennzeichnet, daß man ein Produkt herstellt, das eine biologische Aktivität aufweist, die mit der des nativen Human-IL-2 vergleichbar ist.
